# EUROPEAN PATENT APPLICATION

(11) **EP 2 003 114 A1**
(43) Date of publication of application: **17.12.2008**
(21) Application number: 07740948.0
(22) Date of filing: 28.03.2007
(51) Int. Cl.: C07C 67/38, B01D 3/14, B01D 3/40, C07C 4/10, C07C 5/23, C07C 11/12, C07C 11/22, C07C 69/653, C07B 61/00

(54) **METHYL METHACRYLATE PRODUCTION PROCESS**

(30) Priority: 31.03.2006 JP 2006097937
(71) Applicant: Sumitomo Chemical Company, Limited, Tokyo 104-8260 (JP)
(72) Inventor: MIZUNO, Masahiko, Nara-shi, Nara 630-8114 (JP); SEO, Tateo, Chiba-shi, Chiba 260-0042 (JP); SUZUTA, Tetsuya, Niihama-shi, Ehime 792-0017 (JP)
(74) Representative: Duckworth, Timothy John
(86) International application number: PCT/JP2007/057512
(87) International publication number: WO 2007/114457

(57) **Abstract**

A method of producing methyl methacrylate comprising the following steps:
Thermal decomposition step: a hydrocarbon having 3 or more carbon atoms is thermally decomposed to obtain a decomposed gas having a total content of propyne and propadiene of 2 wt% or more,
Separation step: mixed liquid rich in propyne and propadiene is separated from the decomposed gas obtained in the thermal decomposition step,
Propyne purification step: the mixed liquid rich in propyne and propadiene obtained in the separation step is subjected to extractive distillation, for separation into purified propyne, and crude propadiene containing propadiene as the main component,
Isomerization step: the crude propadiene obtained in the propyne purification step is isomerized in the presence of an isomerization catalyst, to obtain crude propyne containing propyne as the main component, and
Carbonylation step: the purified propyne obtained in the propyne purification step is reacted with carbon monoxide and methanol in the presence of a group VIII metal catalyst system, to produce methyl methacrylate.

## Description

### Technical Field

The present invention relates to a method of producing methyl methacrylate. More particularly, the present invention relates to a method of producing methyl methacrylate, having excellent features such as producing ability on the scale of 100000 tons or more a year and economical. Methyl methacrylate is important as a raw material for synthetic resins and the like.

### Background Art

Japanese Patent Application Laid-Open (JP-A) No. 02-290831 describes a method in which a mixture of propyne and propadiene by-produced in a plant for thermally decomposing a hydrocarbon having 2 to 10 carbons (popular name: ethylene plant)(hereinafter, referred to as ethylene plant in some cases), this plant being carried out for the purpose of producing various petrochemical basic raw materials typically including ethylene, propylene and aromatic hydrocarbons, is taken out by extraction, and this is used as a starting raw material and reacted with carbon monoxide and methanol in the presence of a palladium catalyst to produce methyl methacrylate. This production method is an attractive production method including a small number of steps and capable of producing methyl methacrylate with high yield.

According to investigation of the present inventors, however, plant performance on the scale of 100000 tons or more a year per plant which is economically advantageous in commodity chemicals is usually difficult due to two reasons described below, thus, it is not necessarily economically or industrially advantageous production method.
(1) The content of a mixture of propyne and propadiene in a decomposed gas in naphtha thermal decomposition or ethane thermal decomposition is as extremely small as 23000 to 33000 tons per 1000000 tons of ethylene in the case of naphtha thermal decomposition and 1000 tons per 1000000 tons of ethylene in the case of ethane thermal decomposition, and even if adjacent to an ethylene center of 1000000 tons class, only about 50000 tons of methyl methacrylate can be produced at maximum (PETROCHEMICAL PROCESS p. 29 (The Japan Petroleum Institute ed., Kodansha, published in 2001)).
(2) To collect by transporting mixtures of propyne and propadiene from a plurality of ethylene plants, for increasing propyne feeding amount, is not practical since propyne is a compound containing a triple bond and having a risk of decomposition explosion. Thus, the scale of the methyl methacrylate production plant is limited to 50000 tons a year per plant at maximum as described above.

### DISCLOSURE OF THE INVENTION

Under such circumstances, the present invention has an object of providing a method of producing methyl methacrylate, having excellent features such as producing ability on the scale of 100000 tons or more a year and economical.

The present inventors have intensively studied noticing a method by which the content of a mixture of propyne and propadiene in a decomposed gas can be regulated to 2 wt% or more described in US Patent No. 6333443, and resultantly found that propyne with quality which can be subjected to methyl methacrylate production can be taken out economically advantageously from the above-described decomposed gas, and that methyl methacrylate can be produced economically and industrially advantageously if the propyne taken out is subjected to methyl methacrylate production, leading to completion of the present invention.

That is, the present invention relates to a method of producing methyl methacrylate comprising the following steps:
Thermal decomposition step: a hydrocarbon having 3 or more carbon atoms is thermally decomposed to obtain a decomposed gas having a total content of propyne and propadiene of 2 wt% or more,
Separation step: mixed liquid rich in propyne and propadiene is separated from the decomposed gas obtained in the thermal decomposition step,
Propyne purification step: the mixed liquid rich in propyne and propadiene obtained in the separation step is subjected to extraction distillation, for separation into purified propyne, and crude propadiene containing propadiene as the main component,
Isomerization step: the crude propadiene obtained in the propyne purification step is isomerized in the presence of an isomerization catalyst, to obtain crude propyne containing propyne as the main component, and
Carbonylation step: the purified propyne obtained in the propyne purification step is reacted with carbon monoxide and methanol in the presence of a group VIII metal catalyst system, to produce methyl methacrylate.

### Brief Description of Drawing

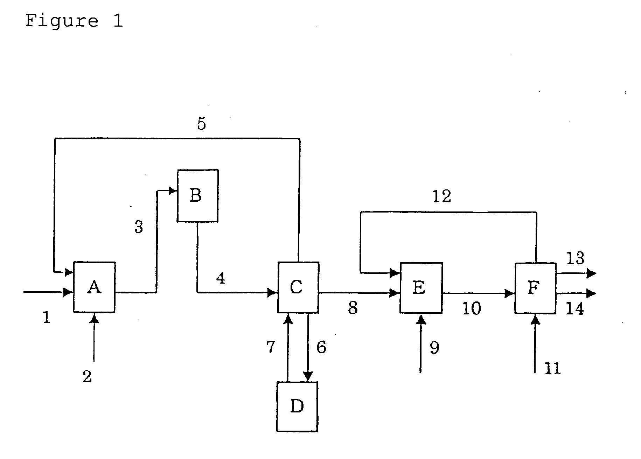

Fig. 1 shows an example of flow in the case of execution of the present invention.

### Explanation of marks

A: thermal decomposition step
B: separation step
C: propyne purification step
D: isomerization step
E: carbonylation step
F: methyl methacrylate purification step

### Modes for Carrying Out the Invention

The present invention includes the following thermal decomposition step, separation step, propyne purification step, isomerization step and carbonylation step.

The thermal decomposition step is a step in which a hydrocarbon having 3 or more carbon atoms is used as a raw material gas and thermally decomposed, to obtain a decomposed gas having a total content of propyne and propadiene of 2 wt% or more.

For example, by thermally decomposing a hydrocarbon having 3 or more carbon atoms according to a method described in US Patent No. 6333443, a decomposed gas having a total content of propyne and propadiene of 2 wt% or more can be obtained. The feeding gas is composed essentially of a raw material gas, a recovery gas and steam. The raw material gas is a hydrocarbon having 3 or more carbon atoms, preferably a hydrocarbon having 3 or 4 carbon atoms, and examples thereof include single gases selected from propane, propylene, butane, 1-butene, 2-butene, isobutane, isobutene and butadiene, or mixed gases composed of two or more of them. The recovery gas is a gas separated from the separation step or propyne purification step. The amount of steam is usually in the range of 0.1 to 5-fold by weight, preferably 0.5 to 2-fold by weight with respect to the total amount of the raw material gas and the recovery gas. The reaction apparatus is a tubular decomposition furnace, and constituted of a raw material pre-heating part, mixed raw material over-heating part and radiation part. The raw material gas and the recovery gas are fed to a pre-heating part and pre-heated. Steam is added to a gas to be discharged from the pre-heating part, and fed to the mixed raw material over-heating part. The temperature in the tube of the radiation part is usually in the range of 400 to 1100°C. The temperature in the tube is not required to be constant, and temperature gradient may exist. The pressure is usually in the range of 0.01 to 1.0 MPa, preferably in the range of 0.05 to 0.3 MPa.

The separation step is a step of separating mixed liquid rich in propyne and propadiene from the decomposed gas obtained in the thermal decomposition step.

Specific examples of this step include embodiments shared with a part, for example, a separation step of a plant (popular name: ethylene plant) for producing hydrogen, methane, ethane, ethylene, propane, propylene, butenes and aromatic hydrocarbon by thermally decomposing a hydrocarbon having 2 to 10 carbon atoms. Here, the hydrocarbon having 2 to 10 carbon atoms include at least one selected from naphtha, butane, propane and ethane.

As further specific and preferable embodiments of the separation step, those including the following steps are exemplified.

First distillation step: the decomposed gas obtained in the thermal decomposition step is cooled, then, fed to a first distillation column, and separated into a fraction 1 composed of a hydrocarbon having 4 or more carbon atoms taken out from the column bottom and a fraction 2 mainly composed of hydrogen and a hydrocarbon having 1 to 3 carbon atoms taken out from the column top,

Second distillation step: the fraction 2 obtained in the first distillation step is fed to a second distillation column, and separated into a fraction 3 mainly composed of a hydrocarbon having 3 carbon atoms taken out from the column bottom and a fraction 4 mainly composed of hydrogen and a hydrocarbon having 1 to 2 carbon atoms taken out from the column top,

Third distillation step: the fraction 3 obtained in the second distillation step is fed to a third distillation column, and separated into a fraction 5 mainly composed of propyne and propadiene taken out from the column bottom and a fraction 6 mainly composed of propylene taken out from the column top.

The decomposed gas derived from the thermal decomposition step is usually composed mainly of hydrogen, methane, ethane, ethylene, acetylene, propane, propylene, propyne, propadiene, butane, butenes or aromatic hydrocarbon. In this case, since hydrogen, ethylene, acetylene, propylene, butenes and aromatic hydrocarbon in the decomposed gas are common to products of an ethylene plant for thermally decomposing a hydrocarbon having 2 to 10 carbon atoms, performed for the purpose of producing various petrochemical basic raw materials, the separation step can also be shared with a separation step of the ethylene plant. Of course, it may also be permissible that the separation step is used solely in a methyl methacrylate plant, and hydrogen, ethylene, acetylene, propylene, butenes, aromatic hydrocarbon and the like are produced as co-products of methyl methacrylate. However, in this case, the production amount of the co-product such as ethylene and the like is correlated with the production amount of methyl methacrylate. Usually, demand for methyl methacrylate is smaller as compared with ethylene and the like, accordingly, when the separation step is used solely in a methyl methacrylate plant, the production amount of ethylene and the like per plant is smaller as compared with an ethylene plant, thus, burden of equipment cost per product in the separation step is larger, leading to economical disadvantage. Therefore, it is more preferable that the separation step is shared with an ethylene plant, and co-products such as ethylene and the like are subjected to an ethylene plant, and only a fraction having a high content of a mixture of propyne and propadiene obtained during the separation step is subjected to a methyl methacrylate plant. For example, the decomposed gas is mixed with a decomposed gas discharged from a decomposition furnace of an ethylene plant, and quenched, then, fed to a first distillation column of the separation step.

The fraction 1 taken out from the column bottom of a first distillation column usually includes butane, butene, aromatic hydrocarbons and water, and these components are subjected to an ethylene plant to give a product of the ethylene plant. The fraction 2 taken out from the column top is usually composed mainly of hydrogen, methane, ethane, ethylene, acetylene, propane, propylene, propyne and propadiene, and propane, propylene, propyne and propadiene are compressed by a compressor for liquefaction, then, subjected to a second distillation column, and separated into a fraction 3 composed mainly of propane, propylene, propyne and propadiene and a fraction 4 composed mainly of hydrogen, methane, ethane, ethylene and acetylene. The fraction 4 is subjected to an ethylene plant like the fraction 1, and used as an active ingredient of the ethylene plant. In contrast, the fraction 3 is subjected to a third distillation column, and separated into a fraction 5 composed mainly of propylene and a fraction 6 composed mainly of propane, propyne and propadiene. Also the fraction 5 is subjected to an ethylene plant as an active ingredient of the ethylene plant like the fraction 1 and the fraction 4. The fraction 6 is subjected to a propyne purification step. The fraction 6 may be separated further precisely into a fraction composed mainly of propane and a fraction rich in propyne and propadiene before only the fraction rich in propyne and propadiene is subjected to a propyne purification step. In this case, the fraction composed mainly of propane is recycled to a thermal decomposition step.

The propyne purification step is a step of subjecting the mixed liquid rich in propyne and propadiene obtained in the separation step to extractive distillation, for separation into purified propyne, and crude propadiene composed mainly of propadiene.

The mixed liquid of the fraction 6 composed mainly of propane, propyne and propadiene obtained in the separation step or the mixed liquid rich in propyne and propadiene prepared by further precisely separating the fraction 6 can be subjected to extractive distillation, to separate purified propyne utilizing a difference in the solubility in the extraction solvent. This mixed liquid to be subjected to extractive distillation may contain propylene. The extraction solvent in the propyne purification step is not particularly restricted providing it manifests a difference in the solubility for propyne, propadiene, propane and propylene. N,N-dimethylformamide is preferable from the standpoints of propyne separation ability, economy, chemical stability and industrial easy availability. When N,N-dimethylformamide is used as the extraction solvent, the solubility is highest for propyne, next for propadiene, third for propylene, and lowest for propane, in this order. For example, when N,N-dimethylformamide is used as the extraction solvent, purified propyne having quality sufficiently satisfactory for subjecting to methyl methacrylate production can be obtained via an extraction step and a propadiene diffusion step described later. With respect to the quality of purified propyne, each content of propane, propylene and propadiene is usually 1 wt% or less, preferably 1000 wt ppm or less, more preferably 100 wt ppm or less.

The extraction step is a step in which the fraction 6 composed mainly of propane, propyne and propadiene obtained in the separation step or the mixed liquid rich in propyne and propadiene prepared by further precisely separating the fraction 6, and N,N-dimethylformamide are subjected to an extractive distillation column, and a mixture composed mainly of propyne, propadiene and N,N-dimethylformamide is obtained from the column bottom and a mixture composed mainly of propane is obtained from the column top.

The propadiene diffusion step is a step in which the mixture composed mainly of propyne, propadiene and N,N-dimethylformamide obtained in the extraction step is subjected to a diffusion column, heat is applied, and crude propadiene composed mainly of propadiene having low solubility is diffused, and liquid composed mainly of propyne and N,N-dimethylformamide is obtained at the column bottom:

The propyne distillation step is a step in which liquid composed mainly propyne and N, N-dimethylformamide, obtained at the column bottom of the diffusion column,are subjected to a distillation column, and purified propyne is obtained from the column top.

The mixture composed mainly of propane generated at the column top of the extraction step is recycled as a recovery gas to a thermal decomposition step. Crude propadiene diffused in the propadiene diffusion step is subjected to an isomerization step. N,N-dimethylformamide remaining at the column bottom of the propyne distillation step may be recycled as it is to an extraction step, or purified by distillation before recycling to an extraction step.

The isomerization step is a step in which the crude propadiene obtained in the propyne purification step is isomerized in the presence of an isomerization catalyst, to obtain crude propyne composed mainly of propyne.

The crude propadiene can be isomerized to thermodynamically stable propyne according to a method described, for example, in JP-A No. 02-290831. The ratio of propyne and propadiene in the feeding liquid is not particularly restricted, and usually, the ratio of propyne/propadiene is 2 or less. The ratio of propyne/propadiene in the isomerized reaction product generated from the reaction vessel depends on the reaction temperature and residence time in the reaction vessel, and is usually 3 or more, preferably 5 or more. The reaction mode is not particularly restricted and includes a liquid phase suspension bed, liquid phase fixed bed, gas phase fixed bed and the like. The crude propyne obtained in the isomerization step is preferably fed to a propyne purification step from the economical standpoint. The isomerization catalyst is preferably an alkali metal or alkali metal oxide supported on alumina from the standpoints of isomerization ability, economy and industrial easy availability.

The carbonylation step is a step in which the purified propyne obtained in the propyne purification step is reacted with carbon monoxide and methanol in the presence of a group VIII metal catalyst system, to produce methyl methacrylate.

By reacting the purified propyne obtained in the propyne purification step with carbon monoxide and methanol in the presence of a group VIII metal catalyst system, methyl methacrylate can be produced. The group VIII metal catalyst system is preferably a catalyst containing palladium element from the standpoint of reaction selectivity. Though the use amount is not particularly restricted, it is usually 1 mol% or less, preferably 0.1 mol% or less, more preferably 0.01 mol% or less with respect to propyne, from the economical standpoint. In contrast, it is, from the standpoint of reactivity, usually 0.00001 mol% or more, preferably 0.0001 mol% or more. A compound containing a phosphorus atom, nitrogen atom, oxygen atom, sulfur atom and the like can be allowed to co-exist as a ligand in the reaction liquid, to control reaction selectivity and catalytic activity. Though the ligand is not particularly restricted, a diaryl (alkyl-substituted 2-pyridyl)phosphine described in JP-A No. 04-215851 is preferably allowed to co-exist from the standpoints of reaction selectivity and catalytic activity. Though the use amount is not particularly restricted, it is usually in the range of 0.1 to 10-fold by mol with respect to a group VIII metal catalyst. Though the solvent is not particularly restricted providing it dissolves propyne, carbon monoxide, methanol and group VIII metal catalyst system, it is preferable, from the standpoint of easiness of recycling, to use, as a solvent, methanol which is also a reaction raw material. The use amount is not particularly restricted. Carbon monoxide can be produced, for example, by a hydrocarbon steam reforming method or partial combustion method, and its production method is not particularly restricted. Carbon dioxide and hydrogen are preferably removed by a pre-treatment since when they are mixed in carbon monoxide, a side reaction occurs in combination. Though the reaction mode is not particularly restricted, it is usually a liquid phase homogeneous reaction system. The reaction temperature is preferably 100°C or lower from the standpoint of suppression of polymerization of methyl methacrylate to be produced.

The production method of the present invention preferably contains a methyl methacrylate purification step described below from the standpoints of the quality of methyl methacrylate and improvement in economy by raw material recycling.

The methyl methacrylate purification step is a step in which the reaction mixture obtained in the carbonylation step is subjected to a gas diffusion operation, distillation operation and/or extraction operation, and unreacted carbon monoxide, propyne and methanol are recovered, and methyl methacrylate is purified.

The reaction liquid generated in the carbonylation step contains mainly methyl methacrylate, methyl crotonate, methanol, propyne, carbon monoxide and group VIII metal catalyst system. Of them, methyl crotonate is a by-product, and it is necessary that the produced component is removed out of the system. It is preferable that methanol, propyne, carbon monoxide and group VIII metal catalyst system are recycled. Though the separation method is not particularly restricted, separation is usually advantageously carried out by distillation utilizing a difference in boiling point or extractive distillation utilizing a difference in solubility in the extraction solvent. For example, first, carbon monoxide as a gas component, and partial propyne not dissolved in the solvent are diffused in a gas diffusion column. Propyne dissolved in the liquid can be diffused partially by accompanying an inert gas fed therein. The accompanying gas includes nitrogen, argon, carbon dioxide, carbon monoxide, methane and the like, and it is preferable to use carbon monoxide which is also a reaction raw material since then separation after diffusion can be omitted. The use amount thereof is preferably an amount at which carbon monoxide is reacted to be converted into methyl methacrylate in one pass. When diffusion of propyne is insufficient at this amount, a single gas selected from nitrogen, argon, carbon dioxide, methane and the like or a mixed gas composed of two or more of them may be fed together. Carbon monoxide and propyne separated are recycled to a carbonylation step. Next, components having higher vapor pressures than that of methyl methacrylate are separated by distillation. The components having higher vapor pressures than that of methyl methacrylate include, for example, propyne and methanol. Thereafter, methyl methacrylate is distilled, and mixed liquid composed mainly of methyl crotonate and group VIII metal catalyst system remains at the column bottom. The mixed liquid composed mainly of methyl crotonate and group VIII metal catalyst system may be discarded, and usually, the group VIII metal is preferably recycled because of high cost. Though the recycling method is not particularly restricted, it is usually a method in which only the metal of an amount corresponding to methyl crotonate generated in the carbonylation step is removed out of the system from the mixed liquid composed mainly of methyl crotonate and group VIII metal catalyst system, and remaining portion of the metal is recycled to the carbonylation step. The temperature of the methyl methacrylate purification step is preferably 100°C or lower from the standpoint of suppression of polymerization of methyl methacrylate. A polymerization inhibitor may also be added. Examples of the polymerization inhibitor include hydroquinone and the like.

### EXAMPLES

The present invention will be illustrated in detail by examples below, but the present invention is not limited to them.

### Example 1

In the case of production of methyl methacrylate via purified propyne to be produced under the following conditions, it can be carried out optimally, for example, according to flow in Fig. 1 and material balance in Tables 1 and 2.

Numbers in the figure correspond to fluid numbers in the tables.
Condition (1): In the thermal decomposition step, propane is used as a raw material, and a decomposed gas having a composition of propyne and propadiene of 2 wt% or more is produced according to US Patent No. 6333443.
Condition (2): The separation step is shared with a separation step of an ethylene plant. Thus, descriptions of hydrocarbon components (fractions) except having 3 carbon atoms are omitted.

34 T/h of propane (fluid number 1), 10.11 T/h of recovery propanes (fluid number 5) and 44.14 T/h of steam (fluid number 2) are fed to a decomposition furnace of the thermal decomposition step (A) and thermally decomposed, to obtain 88.25 T/h of a decomposed gas (fluid number 3) having a total content of propyne and propadiene of 9.3 wt%. The resultant decomposed gas (fluid number 3) is fed to a separation step (B) of an ethylene plant, combined with fluid of the ethylene plant, and first, separated into a fraction 1 having 4 or more carbon atoms taken out from the column bottom and a fraction 2 having 3 or less carbon atoms taken out from the column top in a first distillation column. The resultant fraction 2 having 3 or less carbon atoms is separated into a fraction 3 having 3 carbon atoms taken out from the column bottom and a fraction 4 having 2 or less carbon atoms taken out from the column top in a second distillation column. The fraction 3 is composed of propylene, propane, propyne and propadiene. Subsequently, the fraction 3 is separated into a fraction 5 composed of propylene at the column top and a fraction 6 (fluid number 4) composed of propane, propyne, propadiene and propylene at the column bottom, in a third distillation column. In this process, the flow rate of the fraction 6 (fluid number 4) is 17.83 T/h. Thus obtained fraction 6 (fluid number 4) is purified by carrying out extractive distillation using N,N-dimethylformamide as an extraction solvent in a propyne purification step (C), and separated into 10.11 T/h of recovery propanes (fluid number 5), 17.37 T/h of crude propadiene (fluid number 6) composed mainly of propadiene and 7.69 T/h of purified propyne (fluid number 8). The crude propadiene (fluid number 6) is isomerized with a potassium carbonate supported on alumina catalyst into 17.37 T/h of crude propyne (fluid number 7) in an isomerization step (D), and recycled to the propyne purification step (C). 7.69 T/h of the purified propyne (fluid number 8) obtained in the propyne purification step (C) is subjected to a carbonylation step (E) together with 6.08 T/h of methanol (fluid number 9) and 14.46 T/h of a recycled fraction (fluid number 12) discharged from a methyl methacrylate purification step combining 5.82 T/h of carbon monoxide (fluid number 11) fed to the methyl methacrylate purification step, and reacted in the presence of a palladium catalyst, to obtain 27.8 T/h of a reaction mixture (fluid number 10) containing 20.2 T/h of methyl methacrylate. The resultant reaction mixture (fluid number 10) and 5.82 T/h of carbon monoxide (fluid number 11) were subjected to a methyl methacrylate purification step (F), and separated into 14.46 T/h of recycled fraction (fluid number 12) composed mainly of carbon monoxide, propyne and methanol, 18.75 T/h of purified methyl methacrylate (fluid number 13) and 1.36 T/h of high boiling point fraction (fluid number 14), by gas diffusion and distillation operation.

**Table 1**

| Fluid number | 1 | 2 | 3 | 4 | 5 | 6 | 7 |
|---|---|---|---|---|---|---|---|
| Temperature (°C) | 30 | 224 | 130 | 55 | 18 | -6 | 28 |
| Weight flow rate(T/h) | 34 | 44.14 | 88.25 | 17.83 | 10.11 | 17.37 | 17.37 |
| Weight percentage (wt%) | | | | | | | |
| Methane | | | 10.5 | | | | |
| Hydrogen | | | 1.8 | | | | |
| Ethane | | | 19.1 | | | | |
| Ethylene | | | 1.3 | | | | |
| Propane | 100.0 | | 0.7 | 54.1 | 95.5 | 37.0 | 37.0 |
| Propylene | | | 4.2 | 1.7 | 3.0 | 15.5 | 15.5 |
| Propyne | | | 5.1 | 25.1 | 1.2 | 24.0 | 43.2 |
| Propadiene | | | 3.86 | 19.1 | 0.4 | 23.4 | 4.2 |
| N,N-dimethylformamide | | | | | | | |
| Methanol | | | | | | | |
| Carbon monoxide | | | | | | | |
| Methyl methacrylate | | | | | | | |
| Water | | 100.0 | 50.0 | | | | |
| Inert gas (nitrogen etc.) | | | | | | | |
| Others | | | 3.5 | | | | |
| Total | 100.0 | 100.0 | 100.0 | 100.0 | 100.0 | 100.0 | 100.0 |

**Table 2**

| Fluid number | 8 | 9 | 10 | 11 | 12 | 13 | 14 |
|---|---|---|---|---|---|---|---|
| Temperature(°C) | -24 | 25 | 40 | 49 | -33 to 51 | 69 | 86 |
| Weight flow rate(T/h) | 7.69 | 6.08 | 27.8 | 5.82 | 14.46 | 18.75 | 1.36 |
| Weight percentage (wt%) | | | | | | | |
| Methane | | | | | | | |
| Hydrogen | | | | | | | |
| Ethane | | | | | | | |
| Ethylene | | | | | | | |
| Propane | | | | | | | |
| Propylene | | | | | | | |
| Propyne | 100.0 | | 9.3 | | 17.3 | | |
| Propadiene | | | | | | | |
| N,N-dimethylformamide | | | | | | | |
| Methanol | | 100.0 | 13.4 | | 25.9 | | |
| Carbon monoxide | | | | 92.8 | 36.7 | | |
| Methyl methacrylate | | | 72.7 | | 9.9 | 100.0 | 14.7 |
| Water | | | | | | | |
| Inert gas (nitrogen etc.) | | | 0.4 | 7.2 | 3.6 | | |
| Others | | | 4.2 | | 6.6 | | 85.3 |
| Total | 100.0 | 100.0 | 100.0 | 100.0 | 100.0 | 100.0 | 100.0 |

### Industrial Applicability

According to the present invention, a method of producing methyl methacrylate, having excellent features such as producing ability on the scale of 100000 tons or more a year and economical can be provided.

## Claims

1. A method of producing methyl methacrylate comprising the following steps:
Thermal decomposition step: a hydrocarbon having 3 or more carbon atoms is thermally decomposed to obtain a decomposed gas having a total content of propyne and propadiene of 2 wt% or more,
Separation step: mixed liquid rich in propyne and propadiene is separated from the decomposed gas obtained in the thermal decomposition step,
Propyne purification step: the mixed liquid rich in propyne and propadiene obtained in the separation step is subjected to extractive distillation, for separation into purified propyne, and crude propadiene containing propadiene as the main component,
Isomerization step: the crude propadiene obtained in the propyne purification step is isomerized in the presence of an isomerization catalyst, to obtain crude propyne containing propyne as the main component, and
Carbonylation step: the purified propyne obtained in the propyne purification step is reacted with carbon monoxide and methanol in the presence of a group VIII metal catalyst system, to produce methyl methacrylate.

2. The production method according to Claim 1, wherein the separation step is shared with a separation step of a plant for producing hydrogen, methane, ethane, ethylene, propane, propylene, butenes and aromatic hydrocarbon by thermal decomposition of a hydrocarbon having 2 to 10 carbon atoms.

3. The production method according to Claim 2, wherein the hydrocarbon having 2 to 10 carbon atoms is at least one selected from naphtha, butane, propane and ethane.

4. The production method according to Claim 1, wherein the separation step comprises the following steps:
First distillation step: the decomposed gas obtained in the thermal decomposition step is cooled, then, fed to a first distillation column, and separated into a fraction 1 composed of a hydrocarbon having 4 or more carbon atoms taken out from the column bottom and a fraction 2 mainly composed of hydrogen and a hydrocarbon having 1 to 3 carbon atoms taken out from the column top,
Second distillation step: the fraction 2 obtained in the first distillation step is fed to a second distillation column, and separated into a fraction 3 mainly composed of a hydrocarbon having 3 carbon atoms taken out from the column bottom and a fraction 4 mainly composed of hydrogen and a hydrocarbon having 1 to 2 carbon atoms taken out from the column top,
Third distillation step: the fraction 3 obtained in the second distillation step is fed to a third distillation column, and separated into a fraction 6 mainly composed of propyne and propadiene taken out from the column bottom and a fraction 5 mainly composed of propylene taken out from the column top.

5. The production method according to Claim 1, comprising the following step:
Methyl methacrylate purification step: the reaction mixture obtained in the carbonylation step is subjected to a gas diffusion operation, distillation operation and/or extraction operation, and unreacted carbon monoxide, propyne and methanol are recovered and methyl methacrylate is purified.

6. The production method according to Claim 1, wherein the crude propyne obtained in the isomerization step is fed to a propyne purification step.

7. The production method according to Claim 1, wherein the extraction solvent for the propyne purification step is N,N-dimethylformamide.

8. The production method according to Claim 1, wherein the isomerization catalyst is an alkali metal or alkali metal oxide supported on alumina.

9. The production method according to Claim 1, wherein the group VIII metal catalyst system contains palladium element.
